# EUROPÄISCHE PATENTSCHRIFT

(11) **EP 2 265 568 B1**
(45) Veröffentlichungstag und Bekanntmachung des Hinweises auf die Patenterteilung: **02.11.2011**
(21) Anmeldenummer: 09734735.5
(22) Anmeldetag: 21.04.2009
(51) Int. Cl.: C07C 67/055, C07C 69/01, B01J 8/02, B01J 19/30, B01J 19/32

(54) **VERFAHREN ZUR HERSTELLUNG VON UNGESÄTTIGTEN CARBONSÄUREESTERN**
PROCESS FOR PREPARING UNSATURATED CARBOXYLIC ESTERS
PROCÉDÉ DE PRÉPARATION D'ESTERS INSATURÉS D'ACIDES CARBOXYLIQUES

(30) Priorität: 24.04.2008 DE 102008001366
(43) Veröffentlichungstag der Anmeldung: 29.12.2010
(73) Patentinhaber: Wacker Chemie AG, 81737 München (DE)
(72) Erfinder: WESTERMAYER, Heribert, 84489 Burghausen (DE); WAGNER, Johann, 84489 Burghausen (DE); DAFINGER, Willibald, 94133 Röhrnbach (DE); HOLL, Peter, 84547 Emmerting (DE)
(74) Vertreter: Schuderer, Michael
(86) Internationale Anmeldenummer: PCT/EP2009/054728
(87) Internationale Veröffentlichungsnummer: WO 2009/130211

(56) Entgegenhaltungen:
- EP-A- 1 655 279
- DE-A1- 2 359 286

## Beschreibung

Die Erfindung betrifft ein Verfahren zur Herstellung von ungesättigten Carbonsäureestern mittels Umsetzung von Alkenen mit 2 bis 6 C-Atomen mit Alkancarbonsäuren mit 1 bis 6 C-Atomen, in Gegenwart eines sauerstoffhaltigen Gases, mittels eines kontinuierlichen, homogenen Gasphasenprozess, und in Gegenwart eines heterogenen Edelmetall-Katalysators.

Die Herstellung von ungesättigten Carbonsäureestern mittels Umsetzung von Alkenen mit 2 bis 6 C-Atomen mit Alkancarbonsäuren mit 1 bis 6 C-Atomen, in Gegenwart eines sauerstoffhaltigen Gases, mittels eines kontinuierlichen, homogenen Gasphasenprozesses, und in Gegenwart eines heterogenen Edelmetall-Katalysators ist bereits bekannt. Von besonderer Bedeutung ist die Herstellung von Vinylacetat durch Umsetzung von Ethylen mit Essigsäure und Sauerstoff oder sauerstoffhaltigen Gasen an Festbettkatalysatoren in der Gasphase.

Die Reaktionen erfolgen im allgemeinen bei Drucken von 1 bis 50 bar, vorzugsweise 5 bis 15 bar, und bei Temperaturen von 50 bis 250°C, vorzugsweise 130 bis 200°C. Geeignete Katalysatoren enhalten einen Edelmetallanteil und einen Aktivatoranteil. Der Edelmetallanteil besteht im allgemeinen aus Palladium und/oder dessen Verbindungen. Häufig ist zusätzlich noch Gold und/oder Rhodium oder deren Verbindungen enthalten. Der Aktivatoranteil besteht aus Verbindungen von Elementen der 1. und/oder der 2. Hauptgruppe und/oder Cadmium. Es können auch Rhenium- und/oder Zirkonverbindungen enthalten sein. Diese Komponenten werden im allgemeinen auf Trägermaterialien aufgebracht, beispielsweise Kieselsäure, Aluminiumsilikate, Titanoxid, Zirkonoxid, Siliciumcarbid oder Aluminiumoxid.

Das zur Reaktion eingesetzte Gemisch (Olefin, Alken, Sauerstoff) enthält im allgemeinen einen mehrfachen molaren Überschuß an Olefin. Der Ethylenumsatz ist daher bei der Reaktion nicht vollständig und das nicht umgesetzte Olefin muß im Kreislauf zur Reaktion zurückgeführt werden. Dieses zurückgeführte olefinhaltige Gas wird als Kreisgas bezeichnet. In einem dem Reaktor vorgeschalteten Sättiger (bei der Vinylacetat-Herstellung: Essigsäuresättiger) wird das olefinhaltige Kreisgas (bei der Vinylacetat-Herstellung: ethylenhaltige Kreisgas) mit der entsprechenden Carbonsäure beladen (bei der Vinylacetat-Herstellung: Essigsäure) und anschließend mit Sauerstoff beladen.

Anschließend wird das Reaktionsgemisch in den Reaktor geleitet. Das den Reaktor verlassende heiße Reaktionsgemisch, welches im Falle der Vinylacetat-Herstellung im wesentlichen aus nicht umgesetztem Ethylen, nicht umgesetzter Essigsäure, nicht umgesetztem Sauerstoff, Vinylacetat, Reaktionswasser, Kohlendioxid, sowie mit dem Sauerstoff und Ethylen eingebrachten Inerten (beispielsweise Stickstoff, Ethan, Methan und Argon) besteht, wird, gegebenenfalls unter Vorschaltung einer Entwässerungskolonne, abgekühlt. Der größte Teil der Essigsäure, ein Teil des Vinylacetats und des Wassers wird dabei kondensiert. Das Kondensat wird in nachfolgenden Schritten aufgetrennt, dessen Bestandteile isoliert und die Essigsäure (Rückessigsäure) in den Prozess zurückgeführt. Das unkondensierte Restgas enthält hauptsächlich Ethylen, CO₂ und Inerten und wird nach CO₂-Wäsche und Inertenabtrennung als Kreisgas in den Essigsäuresättiger geführt.

Die Beladung von Kreisgas mit Essigsäure im Essigsäuresättiger hat allerdings den Nachteil, dass es bereits nach kurzen Laufzeiten zur Verschmutzung des Essigsäuresättigers kommt. Bei dem Sättiger handelt es sich im allgemeinen um eine Kolonne in welchem trockenes Kreisgas (ohne Essigsäure und Wasser) zunächst direkt in die Kolonne von unten nach oben geführt wird und Essigsäure dosiert zugeführt wird. Vor allem im unteren Kolonnenbereich kommt es an der Einleitstelle des trockenen und heißen Kreisgases zu Verschmutzungen, welche die Produktionskapazität beeinträchtigen und sogar eine Abstellung der Produktion zur Reinigung auslösen.

Aus der US 6,420,595 B1 ist bekannt, den Sättiger mit einer Destillationskolonne auszustatten, die Verschmutzungen mit dem Kolonnensumpf zu entnehmen und in einer Acid Recovery Unit (A-RU) die Verunreinigungen von der Essigsäure im Vakuum abzutrennen, und letztere in den Sättiger zurückzuführen.

In der US 7,202,377 B1 wird die Bildung von Verschmutzungen im Essigsäuresättiger dem Anteil an Rückessigsäure zugeschrieben, welchen man aus dem kondensierten Anteil des Reaktionsgemisch erhalten hat und der neben Frischessigsäure zur Sättigung des Kreisgases eingesetzt wird. Es wird empfohlen, das Kreisgas über eine der Sättigungskolonne aufgesetzte Rektifikationssektion zu reinigen und die vom Sumpf der Sättigungskolonne abfließende Flüssigkeit in zwei Teilströme aufzuteilen, wobei ein Teilstrom ohne Aufarbeitung zurückgeführt wird, und der andere Teilstrom, nach Abtrennung von Hochsiedern und Polymerisaten in einem Hochtemperatur-Dünnschichtverdampfer, zurückgeführt wird.

Der vorliegenden Erfindung lag die Aufgabe zugrunde ein Verfahren zur Herstellung von ungesättigten Carbonsäureestern zur Verfügung zu stellen, bei welchem die Bildung von Verschmutzungen in dem, dem Reaktor vorgeschalteten Säuresättiger, so wirksam unterbunden wird, dass die aus dem Stand der Technik bekannte, aufwendige Reinigung vermieden werden kann.

Überraschenderweise wurde gefunden, dass durch eine Vorsättigung des Kreisgases, vorzugsweise mit Rückessigsäure, in einem vorgeschalteten Vorsättiger, die Verschmutzung im Säuresättiger drastisch reduziert werden können.

Gegenstand der Erfindung ist ein Verfahren zur Herstellung von ungesättigten Carbonsäureestern mittels Umsetzung von Alkenen mit 2 bis 6 C-Atomen mit Alkancarbonsäuren mit 1 bis 6 C-Atomen, in Gegenwart eines sauerstoffhaltigen Gases und in Gegenwart eines heterogenen Edelmetall-Katalysators mittels eines kontinuierlichen, homogenen Gasphasenprozess in einem Reaktor, wobei eine gasförmige Phase (Kreisgas) im Kreis geführt wird, und das Kreisgas vor dem Eintritt in den Reaktor in einem Säuresättiger mit Alkancarbonsäure beladen wird, **dadurch gekennzeichnet, dass** dem Säuresättiger ein Vorsättiger vorgeschaltet wird, in welchem das Kreisgas mit einer Teilmenge der zur Sättigung eingesetzten Alkancarbonsäuremenge beladen wird, anschließend in den Säuresättiger überführt wird und dort mit der Restmenge an Alkancarbonsäure beladen wird.

Die Erfindung wird am Beispiel der Vinylacetat-Herstellung beschrieben, wobei das Verfahren allgemein zur Herstellung von ungesättigten Carbonsäureestern eingesetzt werden kann. Die Beschreibung ist daher so zu verstehen, dass in dem anschließend beschriebenen Prozeß anstelle von Ethylen allgemein Alkene mit 2 bis 6 C-Atomen eingesetzt werden können, und anstelle von Essigsäure auch andere Alkancarbonsäuren mit 1 bis 6 C-Atomen eingesetzt werden können.

Als Vorsättiger, wie als Sättiger, geeignet sind Gas-Flüssig-Kontakt-Apparaturen allgemein, beispielsweise Kolonnen mit Grid-Packungen, Kolonnen mit Füllkörpern, Kolonnen mit Böden oder anderen Einbauten sowie Venturiwäscher, und im einfachsten Fall eine Rohrleitung. Bevorzugt werden für den Vorsättiger einbautenfreie Quenchen oder Wäscher, beispielsweise Sprüh-, Strahl- oder Venturi-Wäscher.

Das Kreisgas wird vorzugsweise von oben in den Vorsättiger eingeführt. Es kann im Gegenstrom zur zugeführten Essigsäure auch von unten zugeführt werden. Vorzugsweise wird es im Gleichstrom mit der Essigsäure geführt. In einer bevorzugten Ausführungsform werden in dem Sättiger in einer Ebene senkrecht zur vertikalen Achse radial gleichmäßig angeordnete Düsen eingesetzt, über welche die Essigsäure in den Vorsättiger eingesprüht wird. Vorzugsweise wird die Essigsäure von oben eingesprüht.

Das ethylenhaltige Kreisgas tritt im allgemeinen mit einer Temperatur von 100 bis 170°C, vorzugsweise mit 120 bis 150°C, in den Vorsättiger ein. Die Essigsäure wird, abhängig vom Druckniveau des Prozesses, mit einer Temperatur von 90 bis 200°C, vorzugsweise mit 100 bis 150°C, in den Vorsättiger eingeführt. Bei Kontakt mit dem Kreisgas verdampft die Essigsäure und das Kreisgas wird abgekühlt. Die Essigsäure wird vorzugsweise in einem solchen Mengenverhältnis eingeführt, dass diese nicht vollständig verdunstet. Vorzugsweise wird die Vorsättigung so geregelt, dass mindestens 5 Gew.%, besonders bevorzugt 25 bis 75 Gew.-% der bei der Vorsättigung zugeführten Essigsäure im Vorsättiger als Flüssigkeit anfallen.

Zur Vorsättigung werden dem ethylenhaltigen Kreisgas vorzugsweise 20 bis 80 Gew.-%, besonders bevorzugt 50 bis 80 Gew.-% der Essigsäure, bezogen auf die Gesamtmenge an Essigsäure, welche in Vorsättiger und Sättiger zugegeben wird, zugegeben. Mit der Restmenge wird das Kreisgas im Sättiger beladen. Zur Vorsättigung kann Essigsäure aus beliebigen Prozeßschritten eingesetzt werden. Beispielsweise Rückessigsäure, welche aus dem, den Reaktor verlassenden, Reaktionsgemisch zurückgewonnen wurde, oder Rückessigsäure aus der Aufarbeitung von im Prozeß flüssig anfallender Essigsäure (Rückstandsaufarbeitung), Frischessigsäure oder Essigsäure aus dem Pumparound des Sättigers. In einer bevorzugten Ausführungsform wird so vorgegangen, dass zur Vorsättigung, vorzugsweise überwiegend, das heißt > 50 Gew-%, oder ausschließlich, die Rückessigsäure eingesetzt wird, welche aus dem, den Reaktor verlassenden, Reaktionsgemisch zurückgewonnen wurde.

Der im Vorsättiger anfallende Anteil an flüssiger Essigsäure wird aus dem Vorsättiger abgezogen. Vorzugsweise wird der flüssige Anteil aus dem Vorsättiger ganz oder teilweise in den Sumpf des Essigsäuresättigers geleitet. Der flüssige Anteil aus dem Vorsättiger kann auch ganz oder teilweise direkt in die Essigsäureaufarbeitung geführt werden.

Der Essigsäuresättiger ist vorzugsweise als Kolonne ausgeführt; beispielsweise als Füllkörperkolonne oder bevorzugt als Bodenkolonne mit einer Anzahl an Rektifizierböden.

Im erfindungsgemäßen Verfahren wird das vorgesättigte Kreisgas aus dem Vorsättiger in den Essigsäuresättiger überführt. Das Temperaturniveau beträgt nun vorzugsweise 80 bis 140°C. Im Sättiger wird das Kreisgas mit der Restmenge an Essigsäure beladen. Vorzugsweise wird dazu frische Essigsäure (Frischessigsäure) eingesetzt. Es kann auch Frischessigsäure in jedweder Kombination mit dem flüssigen Essigsäureanteil aus dem Vorsättiger und/oder Rückessigsäure, welche aus dem, den Reaktor verlassenden, Reaktionsgemisch und/oder der Rückstandsaufarbeitung zurückgewonnen wurde, eingesetzt werden. Die Zuleitung des ethylenhaltigen, teilweise mit Essigsäure gesättigten, Kreisgasstromes erfolgt vorzugsweise im unteren Viertel, besonders bevorzugt unter dem untersten Boden und oberhalb des Flüssigkeitsspiegels des Essigsäuresättigers. In einer weiteren Ausführungsform kann diesem ethylenhaltigen, mit Essigsäure beladenen Kreisgas aus dem Vorsättiger, vor Eintritt in den Essigsäuresättiger, ein Teilstrom entnommen werden, vorzugsweise bis zu 40 Vol.-%, und dieser Teilstrom oberhalb der Zuführung des Hauptstromes, sozusagen als Bypass, in den Essigsäuresättiger eingeleitet.

Am Boden des Essigsäuresättigers kann Sumpfprodukt entnommen werden. In einer bevorzugten Ausführungsform wird am Boden des Essigsäuresättigers Sumpfprodukt entnommen, aufgewärmt und in den Essigsäuresättiger zurückgeführt. Diese Rückführung kann in einer bevorzugten Ausführung auf mehrere Böden verteilt erfolgen. Über diesen sogenannten Pumparound kann die Temperatur im Essigsäuresättiger und damit die Beladung des Kreisgases mit Essigsäure reguliert werden. Im Falle eines Pumparounds, wird der vorher beschriebene Bypass oberhalb der untersten Zuführung des Pumparounds in den Essigsäuresättiger eingeleitet.

Nach der Beladung mit Essigsäure und der Beladung mit Ethylen und Sauerstoff wird das Kreisgas in den Reaktor überführt.

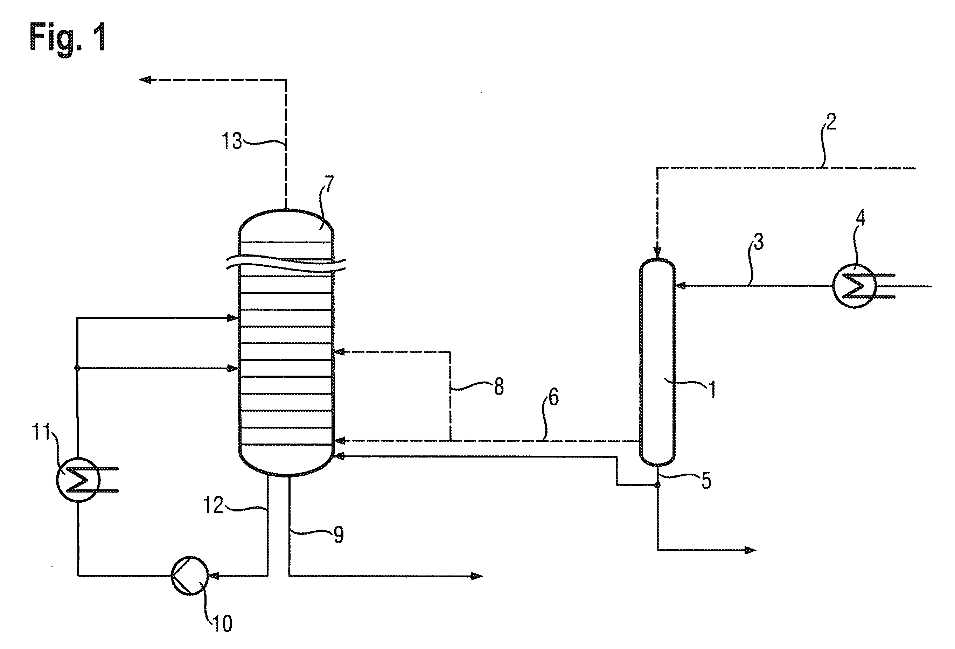

In Figur 1 wird beispielhaft eine schematische Ausführungsform des erfindungsgemäßen Verfahrens dargestellt:

In einen Vorsättiger 1 werden im oberen Drittel über Leitung 2 ethylenhaltiges Kreisgas und über Leitung 3, mit einer Heizeinrichtung 4 vorerwärmte, Essigsäure im Gleichstrom zugeführt. Über Leitung 5 kann dem Vorsättiger flüssiges Sumpfprodukt entnommen werden und ganz oder teilweise zum Essigsäuresättiger 7 oder zur Rückstandsaufarbeitung geführt werden. Das mit Essigsäure beladene, ethylenhaltige Kreisgas wird im unteren Drittel des Vorsättigers über Leitung 6 entnommen und in den Essigsäuresättiger 7 , vorzugsweise in dessen unterem Drittel, überführt.

In der bevorzugten Ausführungsform wird dem ethylenhaltigen, mit Essigsäure beladenen Kreisgas vor Eintritt in den Essigsäuresättiger über Leitung 8 ein Teilstrom entnommen, und dieser Teilstrom oberhalb der untersten Zuführung von Leitung 6 in den Essigsäuresättiger 7 eingeleitet.

Über Leitung 9 kann dem Essigsäuresättiger 7 flüssiges Sumpfprodukt entnommen werden. Über die mit Pumpe 10 und Heizeinrichtung 11 ausgestattete Leitung 12 kann Sumpfprodukt nach Erwärmung in den Essigsäuresättiger 7 zurückgeführt werden (Pumparound).

Das mit Essigsäure gesättigte Kreisgas verläßt den Essigsäuresättiger über Leitung 13.

Bei einer Betriebsweise ohne Vorsättiger mußten in einer Anlage zur Produktion von Vinylacetat in industriellem Maßstab ( ca. 200000 jato VAc) bereits nach ca. 2 bis 3 Monaten die Umpumpmengen und später dann auch die Kreisgasmenge reduziert werden, wegen steigenden Druckverlusten und Fluterscheinungen in der Kolonne, welche auf die Verunreinigung des Essigsäuresättigers zurückzuführen waren. Es traten Probleme beim Flüssigkeitsablauf in der Kolonne auf, die zu Standschwankungen im Sumpf und in Extremfällen zum Versiegen des Sumpfstromes führten. In fortgeschrittenem Zustand ging die Produktion zurück und letzendlich mußten in 3- bis 9-monatigen Abständen Reinigungsabstellungen mit Produktionsausfall durchgeführt werden.

Durch den Einbau des Essigsäurevorsättigers verlängerten sich die Reinigungszyklen auf das 2- bis 3-fache Zeitinterval.

## Patentansprüche

1. Verfahren zur Herstellung von ungesättigten Carbonsäureestern mittels Umsetzung von Alkenen mit 2 bis 6 C-Atomen mit Alkancarbonsäuren mit 1 bis 6 C-Atomen, in Gegenwart eines sauerstoffhaltigen Gases und in Gegenwart eines heterogenen Edelmetall-Katalysators mittels eines kontinuierlichen, homogenen Gasphasenprozess in einem Reaktor, wobei eine gasförmige Phase im Kreis geführt wird, und das Kreisgas vor dem Eintritt in den Reaktor in einem Säuresättiger mit Alkancarbonsäure beladen wird, **dadurch gekennzeichnet, dass** dem Säuresättiger ein Vorsättiger vorgeschaltet wird, in welchem das Kreisgas mit einer Teilmenge der zur Sättigung eingesetzten Alkancarbonsäuremenge beladen wird, anschließend in den Säuresättiger überführt wird und dort mit der Restmenge an Alkancarbonsäure beladen wird.

2. Verfahren nach Anspruch 1, **dadurch gekennzeichnet, dass** zur Vorsättigung 20 bis 80 Gew.-% der Alkancarbonsäure, bezogen auf die Gesamtmenge an Alkancarbonsäure, welche in Vorsättiger und Sättiger zugegeben wird, zugegeben werden.

3. Verfahren nach Anspruch 1 oder 2, **dadurch gekennzeichnet, dass** zur Vorsättigung überwiegend Alkancarbonsäure eingesetzt wird, welche aus dem, den Reaktor verlassenden, Reaktionsgemisch zurückgewonnen wurde.

4. Verfahren nach Anspruch 1 bis 3, **dadurch gekennzeichnet, dass** die Vorsättigung so geregelt wird, dass mindestens 5 Gew.-% der zur Vorsättigung zugeführten Alkancarbonsäure im Vorsättiger als Flüssigkeit anfallen.

5. Verfahren nach Anspruch 1 bis 4, **dadurch gekennzeichnet, dass** das alkenhaltige Kreisgas mit einer Temperatur von 100 bis 170°C in den Vorsättiger eintritt und die Alkancarbonsäure mit einer Temperatur von 90 bis 200°C in den Vorsättiger eintritt.

6. Verfahren nach Anspruch 1 bis 5, **dadurch gekennzeichnet, dass** der Sättiger mit der Restmenge an Alkancarbonsäure beladen wird und dazu frische Alkancarbonsäure eingesetzt wird oder frische Alkancarbonsäure in jedweder Kombination mit dem flüssigen Essigsäureanteil aus dem Vorsättiger und/oder Rückessigsäure, welche aus dem, den Reaktor verlassenden, Reaktionsgemisch und/oder der Rückstandsaufarbeitung zurückgewonnen wurde, eingesetzt wird.

7. Verfahren nach Anspruch 1 bis 6, **dadurch gekennzeichnet, dass** dem alkenhaltigen, mit Alkancarbonsäure beladenen Kreisgas aus dem Vorsättiger, vor Eintritt in den Sättiger, ein Teilstrom entnommen wird, und dieser Teilstrom oberhalb der Zuführung des Hauptstromes als Bypass in den Sättiger eingeleitet wird.

8. Verfahren nach Anspruch 1 bis 7, **dadurch gekennzeichnet, dass** am Boden des Sättigers Sumpfprodukt entnommen wird, und nach Aufwärmen in den Sättiger zurückgeführt wird.

9. Verfahren nach Anspruch 1 bis 8, **dadurch gekennzeichnet, dass** als Alken Ethylen und als Alkancarbonsäure Essigsäure eingesetzt wird.

## Claims

1. Process for preparing unsaturated carboxylic esters by means of reaction of alkenes having 2 to 6 carbon atoms with alkanecarboxylic acids having 1 to 6 carbon atoms, in the presence of an oxygenous gas and in the presence of a heterogeneous noble metal catalyst, by means of a continuous homogeneous gas phase process in a reactor, by circulating a gaseous phase and loading the cycle gas with alkanecarboxylic acid in an acid saturator before entry into the reactor, **characterized in that** upstream of the acid saturator is connected a presaturator in which the cycle gas is loaded with a portion of the amount of alkanecarboxylic acid used for saturation, then is transferred into the acid saturator and loaded there with the remaining amount of alkanecarboxylic acid.

2. Process according to Claim 1, **characterized in that** 20 to 80% by weight of the alkanecarboxylic acid, based on the total amount of alkanecarboxylic acid which is added in presaturator and saturator, is added for presaturation.

3. Process according to Claim 1 or 2, **characterized in that** predominantly alkanecarboxylic acid which has been recovered from the reaction mixture leaving the reactor is used for presaturation.

4. Process according to Claims 1 to 3, **characterized in that** the presaturation is regulated such that at least 5% by weight of the alkanecarboxylic acid supplied to the presaturation is obtained as a liquid in the presaturator.

5. Process according to Claims 1 to 4, **characterized in that** the alkene-containing cycle gas enters the presaturator at a temperature of 100 to 170°C and the alkanecarboxylic acid enters the presaturator with a temperature of 90 to 200°C.

6. Process according to Claims 1 to 5, **characterized in that** the saturator is loaded with the remaining amount of alkanecarboxylic acid and fresh alkanecarboxylic acid is used for this purpose, or fresh alkanecarboxylic acid is used in any combination with the liquid acetic acid component from the presaturator and/or returned acetic acid which has been recovered from the reaction mixture leaving the reactor and/or the residue work-up.

7. Process according to Claims 1 to 6, **characterized in that** a substream is withdrawn from the alkene-containing cycle gas laden with alkanecarboxylic acid from the presaturator, before entry into the saturator, and this substream is introduced into the saturator as a bypass above the feed of the main stream.

8. Process according to Claims 1 to 7, **characterized in that** bottom product is withdrawn at the bottom of the saturator and is recycled into the saturator after heating.

9. Process according to Claims 1 to 8, **characterized in that** the alkene used is ethylene and the alkanecarboxylic acid is acetic acid.

## Revendications

1. Procédé de fabrication d'esters insaturés d'acides carboxyliques par mise en réaction d'alcènes de 2 à 6 atomes C avec des acides alcanecarboxyliques de 1 à 6 atomes C en présence d'un gaz contenant de l'oxygène et en présence d'un catalyseur de métal noble hétérogène par un procédé continu en phase gazeuse homogène dans un réacteur, une phase gazeuse étant mise en circulation dans un circuit et le gaz circulaire étant chargé avec un acide alcanecarboxylique dans un saturateur d'acide avant l'entrée dans le réacteur, **caractérisé en ce qu'**un présaturateur est connecté en amont du saturateur d'acide, dans lequel le gaz circulaire est chargé avec une partie de la quantité d'acide alcanecarboxylique utilisée pour la saturation, puis il est transféré dans le saturateur d'acide et y est chargé avec la quantité restante d'acide alcanecarboxylique.

2. Procédé selon la revendication 1, **caractérisé en ce que** 20 à 80 % en poids de l'acide alcanecarboxylique, par rapport à la quantité totale d'acide alcanecarboxylique ajoutée dans le présaturateur et le saturateur, est ajouté pour la présaturation.

3. Procédé selon la revendication 1 ou 2, **caractérisé en ce que** principalement de l'acide alcanecarboxylique qui a été récupéré du mélange réactionnel quittant le réacteur est utilisé pour la présaturation.

4. Procédé selon les revendications 1 à 3, **caractérisé en ce que** la présaturation est ajustée de manière à ce qu'au moins 5 % en poids de l'acide alcanecarboxylique introduit pour la présaturation s'accumule sous forme liquide dans le présaturateur.

5. Procédé selon les revendications 1 à 4, **caractérisé en ce que** le gaz circulaire contenant l'alcène entre à une température de 100 à 170 °C dans le présaturateur et l'acide alcanecarboxylique entre à une température de 90 à 200 °C dans le présaturateur.

6. Procédé selon les revendications 1 à 5, **caractérisé en ce que** le saturateur est chargé avec la quantité restante d'acide alcanecarboxylique et, pour cela, on utilise de l'acide alcanecarboxylique frais ou de l'acide alcanecarboxylique frais en une combinaison quelconque avec la fraction d'acide acétique liquide issue du présaturateur et/ou l'acide acétique recyclé qui a été récupéré du mélange réactionnel quittant le réacteur et/ou du traitement du résidu.

7. Procédé selon les revendications 1 à 6, **caractérisé en ce qu'**un courant partiel est soutiré du gaz circulaire contenant l'alcène, chargé avec l'acide alcanecarboxylique, issu du présaturateur avant l'entrée dans le saturateur, et ce courant partiel est introduit dans le saturateur sous la forme d'une dérivation au-dessus de l'alimentation du courant principal.

8. Procédé selon les revendications 1 à 7, **caractérisé en ce qu'**un produit de fond est soutiré au fond du saturateur et recyclé dans le saturateur après réchauffement.

9. Procédé selon les revendications 1 à 8, **caractérisé en ce que** de l'éthylène est utilisé en tant qu'alcène et de l'acide acétique en tant qu'acide alcanecarboxylique.
